Europäisches Patentamt

European Patent Office

Office européen des brevets

(19)

(11) **EP 1 035 101 B1**

## (12) EUROPÄISCHE PATENTSCHRIFT

(45) Veröffentlichungstag und Bekanntmachung des Hinweises auf die Patenterteilung:
**21.11.2001 Patentblatt 2001/47**

(51) Int Cl.[7]: **C07C 51/21**, C07C 51/42, C07C 53/08, C07C 53/02

(21) Anmeldenummer: **00102291.2**

(22) Anmeldetag: **17.02.2000**

(54) **Verfahren und Vorrichtung zur Herstellung von gesättigten Carbonsäuren mit ein bis vier C-Atomen**

Process and apparatus for the production of carboxylic acids with one to four carbon atoms

Procédé et dispositif pour la production d'acides carboxyliques à un à quatre atomes de carbone

(84) Benannte Vertragsstaaten:
**AT BE CH CY DE DK ES FI FR GB GR IE IT LI LU MC NL PT SE**

(30) Priorität: **11.03.1999 DE 19910866**

(43) Veröffentlichungstag der Anmeldung:
**13.09.2000 Patentblatt 2000/37**

(73) Patentinhaber: **Consortium für elektrochemische Industrie GmbH**
**81379 München (DE)**

(72) Erfinder:
• **Rüdinger, Christoph, Dr.**
**82319 Starnberg (DE)**

• **Eberle, Hans-Jürgen, Dr.**
**81477 München (DE)**
• **Hallmann, Michael**
**5122 Ach (AT)**

(74) Vertreter: **Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
WO-A-98/23371          DE-A- 1 921 503
DE-A- 19 823 088

**Beschreibung**

Gegenstand der Erfindung ist ein Verfahren zur Herstellung von gesättigten Carbonsäuren mit ein bis vier C-Atomen, sowie eine Vorrichtung zur Durchführung des Verfahrens.

Es ist bekannt, daß Essigsäure durch Gasphasenoxidation von $C_4$-Kohlenwasserstoffen in Gegenwart eines Katalysators hergestellt werden kann. Die meisten Beschreibungen sehen vor, das Reaktionsgasgemisch einmal über den Katalysator zu leiten, die gebildete Essigsäure durch Kondensation abzutrennen und das verbleibende Gas zu verwerfen. In der US-A 3,917,682 wird beispielsweise ein Vorgehen beschrieben, bei dem Essigsäure durch Butenoxidation in Gegenwart eines Ti/V-Katalysators mit hohem Rutilanteil gewonnen wird, wobei die Essigsäure durch Partialkondensation des Reaktionsgemisches isoliert wird, der verbleibende Rest des Reaktionsgases wird nicht zurückgeführt. Derartige Verfahren müssen bereits bei einmaligem Reaktordürchgang einen hohen Butenumsatz erreichen, was nur mit geringen Ausbeuten oder geringen Raum-Zeit-Leistungen gelingt. Auf dieser Verfahrensbasis konnte deshalb bisher noch kein wirtschaftlich befriedigendes Verfahren gefunden werden.

Aus der US-A 4,146,734 ist bekannt, die Gasphasenoxidation von Buten zu Essigsäure in Gegenwart eines Lanthaniden-Verbindungen enthaltenden Katalysators durchzuführen. Ein Verfahren zur Isolierung der Essigsäure und weiterer während der Gasphasenoxidation gebildeter Wertstoffe wird nicht angegeben.

Die DE-A 2149752 und die DE-A 1279011 beschreiben Verfahren zur katalytischen Gasphasenoxidation von Buten zu Essigsäure in Gegenwart von speziellen Katalysatoren. Nachteilig bei dieser Verfahrensweise ist, daß bei der dort angegebenen Rückführung des nicht kondensierbaren Anteils des Reaktionsgases der Anteil der als Wertstoff anfallenden Ameisensäure zerfällt. In der DE-A 1921503 wird auf die Möglichkeit hingewiesen bei der Essigsäureherstellung mittels katalytischer Gasphasenoxidation von Buten den nicht reagierten Anteil des Reaktionsgemisches in den Reaktor zurückzuführen, es wird aber ausdrücklich auf die Unwirtschaftlichkeit eines Kreisgasverfahrens verwiesen.

Das von den Chemischen Werken Hüls zum Pilotmaßstab entwickelte, und in verschiedenen Publikationen (R.P. Lowry, A. Aguilo, Hydrocarbon Processing, 10, (1974), 103; PEP Report No. 37A (1973)) beschriebene Verfahren sieht die direkte, unbehandelte Rückführung von 4/5 des den Reaktor verlassenden Gasgemisches vor. Bei dieser Ausführungsform wird das Reaktionsprodukt teilweise ohne Abtrennung der Säuren im Kreis geführt und nur ein Teil zur Isolierung der Essigsäure abgetrennt. Bei diesem Verfahren kommt es zu einer deutlichen Anreicherung von organischen Säuren im Reaktionsgas, wodurch sowohl Essigsäure als auch Ameisensäure in nicht befriedigender Ausbeute erhalten werden.

Aus WO-A 9823371 ist ein Verfahren zur Herstellung von Essigsäure durch Gasphasenoxidation von ungesättigten $C_4$-Kohlenwasserstoffen unter Verwendung eines Schalenkatalysators aus einem Trägerkörper und einer auf die äußere Oberfläche des Trägerkörpers aufgebrachten katalytisch aktiven Mischoxidmasse aus der Gruppe Titandioxid, Zirkoniumdioxid, Zinndioxid, Aluminiumoxid und Vanadiumpentoxid bekannt. Nach der Umsetzung wird die gebildete Essigsäure durch Abkühlen und Niederschlagen oder durch Absorption in einem geeigneten Lösungsmittel abgetrennt.

Die DE-A 19823052 beschreibt ein Verfahren zur Herstellung von Essigsäure durch Gasphasenoxidation von gesättigten $C_4$-Kohlenwasserstoffen und deren Gemische mit ungesättigten $C_4$-Kohlenwasserstoffen unter Verwendung eines Schalenkatalysators der aus einem inerten unporösen Trägerkörper und einer auf die äußere Oberfläche des Trägerkörpers aufgebrachten katalytisch aktiven Mischoxidmasse aus Titandioxid und Vanadiumpentoxid besteht. Dabei wird ein Gasgemisch aus einem sauerstoffhaltigen Gas und $C_4$-Kohlenwasserstoffen mit Wasserdampf bei einer Temperatur von 100°C bis 400°C, und einem Überdruck von $1,2*10^5$ bis $51*10^5$ Pa an dem Schalenkatalysator umgesetzt. Ein Verfahren zur Isolierung der Essigsäure und weiterer während der Gasphasenoxidation gebildeter Wertstoffe wird nicht angegeben.

Die DE-A 19823088 beschreibt ein Verfahren zur Herstellung von gesättigten Carbonsäuren mit 1 bis 4 C-Atomen durch Gasphasenoxidation in Gegenwart von gesättigten und/oder ungesättigten C4-Kohlenwasserstoffen, einem sauerstoffhaltigen Gas und Wasserdampf in Anwesenheit von zumindest einem Katalysator. Bei diesem Verfahren wird das Reaktionsausgangsgas in einem Reaktionsgaskreislauf zum Teil zurückgeführt, wobei der Reaktionsgaskreislauf so ausgeführt wird, daß dem Reaktionsausgangsgas ein Teil der bei der Gasphasenoxidation entstandenen organischen Säuren entzogen wird. Zur Abtrennung der Rohsäure werden aber nur unwirtschaftliche Verfahren wie beispielsweise die partielle Kondensation des Gasgemisches, oder auch Verfahren durch Rektifikation, gegebenenfalls unter Zusatz von Hilfsstoffen (z.B. Extraktivrektifikation), angegeben.

Es bestand daher die Aufgabe ein Verfahren zur Herstellung von gesättigten Carbonsäuren mit ein bis vier C-Atomen, insbesondere Essigsäure, durch Gasphasenoxidation von gesättigten und/oder ungesättigten $C_4$-Kohlenwasserstoffen zu finden, welches hohe Säureausbeuten liefert und bei dem die Nebenprodukte als Wertstoffe anfallen.

Es wurde überraschend gefunden, daß sich die Herstellung von gesättigten Carbonsäuren mit ein bis vier C-Atomen durch Gasphasenoxidation von gesättigten und/oder ungesättigten $C_4$-Kohlenwasserstoffen mit besonders hohen Ausbeuten durchführen läßt, wenn ein durch eine wäßrige Gegenstromwäsche von Säuren überwiegend befreiter Teilstrom des Reaktionsausgangsgasgemisches an den Reaktoreingang zurückgeführt wird.

Gegenstand der Erfindung ist ein Verfahren zur Herstellung von gesättigten Carbonsäuren mit ein bis vier C-Atomen durch Gasphasenoxidation bei einer Reaktionstemperatur von 100°C bis 400°C und bei Drücken von $1,2*10^5$ bis $51*10^5$ Pa, in Gegenwart von gesättigten oder ungesättigten $C_4$-Kohlenwasserstoffen und deren Gemischen, einem sauerstoffhaltigem Gas und Wasserdampf, in Anwesenheit von zumindest einem Katalysator, wobei das Reaktionsausgangsgas in einem Reaktionsgaskreislauf zum Teil zurückgeführt wird und dabei durch einen Abtrennschritt die Säurekonzentration im zurückgeführten Anteil verringert wird, dadurch gekennzeichnet, daß die Rohsäure aus dem Reaktionsausgangsgas durch eine Gegenstromwäsche abgetrennt wird.

Bei dem erfindungsgemäßen Verfahren wird der Reaktionsgaskreislauf so ausgeführt, daß dem Reaktionsausgangsgas, also entweder dem den Reaktor verlassenden oder dem zurückgeführten Gasgemisch, ein Teil der organischen Säuren, primär Essigsäure und Ameisensäure, über eine Gegenstromwäsche mit einem geeigneten Lösungsmittel, bevorzugt Wasser, entzogen wird. Die Abtrennung wird dabei so ausgeführt, daß der Partialdruck dieser Säuren am Reaktoreingang niedrig bleibt, nicht umgesetzte $C_4$-Kohlenwasserstoffe und weiter zu Essigsäure umsetzbare Zwischenprodukte wie beispielsweise Acetaldehyd, Aceton, Methylethylketon und 2-Butanol aber größtenteils im Kreisgas verbleiben und zum Reaktoreingang zurückgeführt werden.

Als geeignete Lösungsmittel für die Gegenstromwäsche werden bevorzugt Verbindungen der Gruppe enthaltend Diphenyloxid, Diphenyl, aromatische- und aliphatische Ketone und Ether, Phthalsäure und Phtalsäurederivate, Phthalid, aliphatische Dicarbonsäuren, Adipinsäure und Adipinsäurederivate, Maleinsäure und Maleinsäurederivate, Carboxyessigsäuren, Benzoesäure und Benzoesäurederivate, Lactone, Propylencarbonat, Dialkylcarbonate, Trialkylphosphate, Trialkylamine, Sulfolan und Sulfolanderivate, Alkylpyrollidone, niedermolekulare d.h. flüssige Polymere bzw. Oligomere, Polyvinylate, Polyacrylate, Polyether, Polyketone, Wasser oder Mischungen solcher Verbindungen verwendet. Besonders bevorzugtes Lösungsmittel ist Wasser.

Ein gutes Lösungsmittel muß folgende Eigenschaften besitzen: Gute Selektivität bezüglich der Wasser/Säure Trennung; hohe Affinität für organische Säuren (hoher Verteilungskoeffizient) um die benötigte Absorptionsmittelmenge gering zu halten; geringe Flüchtigkeit unter den Absorptionsbedingungen um die Verluste ins Kreisgas gering zu halten; die ins Kreisgas übergehenden Anteile des Absorptionsmittels dürfen die katalytische Oxidationsreaktion nicht negativ beeinflussen; der Schmelzpunkt muß deutlich unterhalb der Absorptionstemperatur legen; es darf unter den Absorptions/Desorptionsbedingungen und Regenerationsbedingungen keine chemischen Reaktionen eingehen.

Die Wäsche des Reaktionsgases bei der erfindungsgemäßen Gegenstromabsorption wird vorteilhaft so ausgeführt, daß das Reaktionsgas eine oder mehrere Vorrichtungen aus der Gruppe enthaltend Riesel- bzw. Sprühturm, einen Absorber mit bewegten Einbauten, beispielsweise einen Rotationsabsorber, einen Absorptionsturm mit Füllkörperschüttung, einen Absorber mit Einbauten in Form von Trennböden, beispielsweise Glockenböden, Ventilböden, Siebböden, Gitterböden oder Kombinationen daraus, oder einen Absorber mit geordneten Packungen durchströmt und dazu im Gegenstrom der Absorber mit Wasser durchströmt wird. Der Wasserdampfgehalt des Gasstroms, das den Absorber verläßt, wird über die am Absorberausgang herrschende Temperatur und den Betriebsdruck festgelegt. Die Temperatur wird über die aus dem Absorber abgeführte Wärmemenge und die Menge und Temperatur des Waschwasserstromes festgelegt und beträgt im allgemeinen 50°C bis 200°C. Der verbleibende Säuregehalt im Gasstrom der den Absorber verläßt, wird über Druck und Temperatur, die Trennstufenzahl des Absorbers und die zugeführte Absorbtionsmittelmenge (Wassereinspeisung) festgelegt. Im allgemeinem wird das Verfahren so ausgeführt, daß durch die Gegenstromwäsche die Restsäurekonzentration des wieder in den Reaktor zurückgeführten Gasstroms auf 0,01 bis 6 Vol.-% reduziert wird.

Neben Essigsäure und Ameisensäure fallen als weitere Wertstoffe Propionsäure, Maleinsäure/Maleinsäureanhydrid und Acrylsäure an. Das bei der Konzentrierung und Reinigung der Rohsäure anfallende Wasser wird teilweise, gegebenenfalls nach einer chemisch und/oder physikalischen Behandlung, in die Gegenstromabsorption zurückgespeist, so daß beim gesamten Prozeß nahezu kein Abwasser anfällt.

Die abgetrennte Rohsäure wird mit einem oder mehreren geeigneten üblichen Verfahren der Gruppe enthaltend Flüssig-Flüssig-Extraktion, Extraktivrektifikation, Azeotroprektifikation, Rektifikation und Membrantrennverfahren entwässert und gereinigt. Die bei der Konzentrierung und Reinigung der Rohsäure anfallenden Leichtsieder können in die Gegenstromwäsche zurückgeführt werden. Die vor einer weiteren Auftrennung der Rohsäure in ihre Reinsubstanzen abgetrennten Leichtsieder können ebenfalls, alleine oder zusammen mit Leichtsiedern aus der Reinigung und Konzentrierung ganz oder teilweise in die Gegenstromwäsche zurückgeführt werden.

Das erfindungsgemäße Verfahren eignet sich vorzugsweise zur Herstellung von Essigsäure und Ameisensäure, besonders bevorzugt zur Herstellung von Essigsäure. Ein wesentlicher Vorteil der erfindungsgemäßen Vorgehensweise besteht darin, daß bei der Herstellung von Essigsäure, die dabei entstehenden Nebenprodukte als Wertstoffe, vor allem in Form von Ameisensäure, anfallen.

Die erfindungsgemäße Abtrennung der organischen Säuren durch Gegenstromabsorption, beispielsweise durch Wasser, hat gegenüber der Kondensation mehrere Vorteile. Zum einen wird dadurch eine höhere Selektivität erreicht, andererseits wird der benötigte Energieaufwand verringert. Weiterhin entsteht eine höhere passive Sicherheit im Hinblick auf die Explosionsgefahr und die gesamte Prozeßführung läßt sich dadurch einfacher gestalten.

Bei den im erfindungsgemäßen Verfahren eingesetzten gesättigten oder ungesättigten Kohlenwasserstoffen mit vier Kohlenstoffatomen handelt es sich um Verbindungen aus der Gruppe umfassend n-Butan, i-Butan, t-Butan, 1-Buten, cis-2-Buten, trans-2-Buten, Isobuten, 1,3-Butadien. Bevorzugt sind n-Butan und die Buten-Isomere 1-Buten, trans-2-Buten und cis-2-Buten sowie Gemische, die diese Verbindungen in hohen Anteilen enthalten. Bei.dem erfindungsgemäßen Verfahren kann die $C_4$-Kohlenwasstofffraktion auch lineare und/oder verzweigte und/oder cyclische Kohlenwasserstoffe mit mehr oder weniger als vier Kohlenstoffatomen wie beispielsweise Methan, Ethan, Ethen, Propen, Propan, Pentane, Pentene, Pentadiene, Cyclopentan, Cyclopenten, Cyclopentadien, Methylcyclopentan enthalten. Ebenso- können Alkohole, Aldehyde, Ether, Ketone und Ester mit 1 bis 8 Kohlenstoffatomen anwesend sein. Bevorzugt werden billige Rohstoffgemische aus der Petrochemie wie "$C_4$-Schnitt" (überwiegender Anteil an Butadien und i-Buten), "Raffinat 1" (überwiegender Anteil an i-Buten und n-Butenen) und "Raffinat 2" (überwiegender Anteil an Butanen, 1-Buten und 2-Butenen) als Ausgangsmaterial oder Mischungen die solche Kohlenwasserstoffe enthalten. Diese können gegebenenfalls nach einer Vorbehandlung z.B. einer Reinigung bzw. Hydrierung verwendet werden.

Die Reaktionstemperatur der Gasphasenoxidation beträgt im allgemeinen 100°C bis 400°C, vorzugsweise 150°C bis 250°C, besonders bevorzugt 180°C bis 230°C. Die Reaktion wird im allgemeinen bei Drücken zwischen $1,2*10^5$ und $51*10^5$ Pa, bevorzugt zwischen $4*10^5$ und $41*10^5$ Pa besonders bevorzugt zwischen $9*10^5$ und $17*10^5$ Pa durchgeführt.

Als sauerstoffhaltiges Gas kann Luft, mit Sauerstoff angereicherte Luft und bevorzugt reiner Sauerstoff verwendet werden. Bei dem erfingungsgemäßen Verfahren kann aber auch ein Inertgas wie beispielsweise Stickstoff anwesend sein.

Der Volumenanteil von Wasserdampf des dem Reaktor zugeführten Reaktoreingangsgases beträgt im allgemeinen 5 bis 80 Vol.-%, vorzugsweise 5 bis 40 Vol.-%, besonders bevorzugt 5 bis 30 Vol.-% Wasserdampf.

Der Anteil an Buten im Reaktionsgas, gemessen am Reaktoreingang, welches allein oder im Gemisch mit weiteren $C_4$-Kohlenwasserstoffen als Edukt vorliegen kann, beträgt 1 bis 10 Vol.-%, vorzugsweise 1,5 bis 3,5 Vol.-%. Der Anteil an Butan im Reaktionsgas, gemessen am Reaktoreingang, welches ebenfalls allein oder im Gemisch mit weiteren $C_4$-Kohlenwasserstoffen als Edukt vorliegen kann, beträgt 5 bis 80 Vol.-%, vorzugsweise 5 bis 60 Vol.-%, besonders bevorzugt 10 bis 50 Vol.-%.

Der Sauerstoffgehalt des dem Reaktor zugeführten Gasstroms beträgt 1 bis 35 Vol.-%, vorzugsweise 2 bis 20 Vol.-%, besonders bevorzugt 3 bis 12 Vol.-%.

Gegebenenfalls kann ein Inertgasanteil von 0 bis 25 Vol.-% zugeführt werden. Der Anteil an Kohlenoxiden und weiteren Reaktionsnebenprodukten im Reaktoreingangsgas hängt von der Reaktionsführung und Säureabtrennung ab und beträgt im allgemeinen 10 bis 80 Vol.-%, bevorzugt 15 bis 65 Vol.-%. Die Anteile in Vol.-% der einzelnen Bestandteile des Reaktoreingangsgases addieren sich dabei jeweils auf 100 Vol.-%.

Der rückgeführte Gasmassenstrom beträgt im allgemeinen zwischen dem 1-fachen und dem 100-fachen des frisch zugeführten Eduktmassenstroms, bevorzugt zwischen dem 5-fachen und 80-fachen, besonders bevorzugt zwischen dem 10 bis 40-fachen.

Als Katalysatoren für das erfindungsgemäße Verfahren eignen sich alle Katalysatoren die allgemein für die partielle Oxidation von gesättigten und/oder ungesättigten $C_4$-Kohlenwasserstoffen zu Essigsäure beschrieben sind. Bevorzugt sind Mischoxidkatalysatoren die Vanadiumoxide enthalten, besonders bevorzugt sind Schalenkatalysatoren wie sie in DE-A 19649426 beschrieben sind. Die DE-A 19649426, deren diesbezügliche Offenbarung Teil dieser Anmeldung sein soll, wird hiermit unter Bezugnahme eingeschlossen (incorporated by reference). Dabei handelt es sich um einen Schalenkatalysator bestehend aus einem inerten unporösen Trägerkörper und einer auf die äußere Oberfläche des Trägerkörpers aufgebrachten katalytisch aktiven Mischoxidmasse, welche a) ein oder mehrere Oxide aus der Gruppe Titandioxid, Zirkoniumdioxid, Zinndioxid, Aluminiumoxid und b) 0,1 bis 1,5 Gew.-%, bezogen auf das Gewicht der Komponente a) und pro $m^2/g$ spezifischer Oberfläche der Komponente a), Vanadiumpentoxid enthält.

Als zusätzliche Komponente a) können noch ein oder mehrere Oxide aus der Gruppe Bor, Silicium, Hafnium, Niob, Wolfram, Lanthan und Cer enthalten sein. Bei der Dotierung der Komponente a) mit den genannten Oxiden sind diese im allgemeinen in einer Menge von 1 bis 30 Gew.-%, bezogen auf das Gesamtgewicht der Komponente a), enthalten.

Bei der Komponente b) kann gegebenenfalls ein Teil des Vanadiumpentoxids, vorzugsweise 10 bis 90 Gew.-%, durch ein oder mehrere Oxide von Molybdän, Chrom und Antimon ersetzt werden. Gegebenenfalls können als zusätzliche Komponente b) noch ein oder mehrere Oxide von Alkalimetallen, Elementen der 5. und 6. Hauptgruppe des Periodensystems der Elemente (PSE) und der Übergangsmetalle enthalten sein. Im allgemeinen beträgt die Menge dieser Dotierstoffe 0,005 bis 15 Gew.-%, berechnet als Oxide und bezogen auf das Gesamtgewicht der Komponente b).

Bevorzugt werden Zusammensetzungen mit hoher Oberfläche der Komponente a) von 40 bis 300 $m^2/g$, wobei gegebenenfalls noch Zinn- Niob- oder Wolframoxid enthalten sein können, und mit einer Komponente b), welche mit Mo, und/oder Cr, und/oder Sb und/oder Au dotiert ist.

Die katalytisch aktive Mischoxidmasse kann gegebenenfalls noch 10 bis 50 Gew.-%, bezogen auf das Gesamtgewicht

EP 1 035 101 B1

der katalytisch aktiven Mischoxidmasse, inerte Verdünnungsmittel aus der Gruppe umfassend Siliciumdioxid, Siliciumcarbid und Graphit enthalten.

Die katalytisch aktive Mischoxidmasse ist in einem Anteil von 1 bis 40 Gew.-%, vorzugsweise 5 bis 25 Gew.-%, jeweils bezogen auf das Gesamtgewicht aus Trägerkörper und aktiver Masse, als Schale auf die äußere Oberfläche des Trägerkörpers aufgebracht.

Die Schichtdicke beträgt im allgemeinen 10 bis 2000 μm, vorzugsweise 100 bis 1000 μm. Der Schalenkatalysator kann auch mehrere, sich in der Zusammensetzung unterscheidende, Schichten enthalten. Es können auch ein oder mehrere Bestandteile der Aktivkomponente a) und b) in unterschiedlicher Konzentration in den einzelnen Schichten enthalten sein.

Geeignete Materialien für den inerten, unporösen Trägerkörper sind alle unter den Betriebsbedingungen der Gasphasenoxidation sich inert verhaltenden und über den Betriebszeitraum stabilen unporösen Materialien. Beispiele hierfür sind Steatit, Duranit, Siliciumcarbid, Magnesiumoxid, Siliciumoxid, Silikate, Aluminate, Metalle wie Edelstahl, sowie gegebenenfalls Mischungen aus diesen Stoffen. Bevorzugtes Material ist keramisches Material, beispielsweise Steatit. Die Formgestalt des inerten, unporösen Trägerkörpers ist beliebig. Beispiele für geeignete Formen sind Kugeln, Zylinder, Quader, Tori, Sättel, Spindeln, Wendeln. Die Grundkörper können auch eine oder mehrere Ausnehmungen wie Mulden, Rillen, Löcher, oder auch abstehende Teile wie Zapfen, Spitzen, Stege besitzen. Weitere Beispiele sind Ringe, Ringsegmente, Steg-Ringe, durchbohrte Kugeln, Kugelsegmente. Ebenfalls als Träger geeignet sind geordnete Packungen wie Monolithe oder Kreuzkanalstrukturen. Bevorzugt sind Trägerformen mit möglichst hoher geometrischer Oberfläche pro Volumen, beispielsweise Ringe.

Die Abmessungen der Trägerkörper sind durch die Reaktoren zur Gasphasenoxidation vorgegeben. Im allgemeinen haben die Formkörper eine Länge bzw. einen Durchmesser von 2 bis 20 mm. Die Wanddicke, beispielsweise im Fall von Ringen oder Hohlzylindern, beträgt zweckmäßigerweise 0,1 bis 4 mm.

Als Reaktor können Ausführungen verwendet werden, die sich für die Durchführung von Oxidationsreaktionen in der Gasphase eignen und in der Lage sind die hohe Reaktionswärme ohne übermäßige Erwärmung des Reaktionsgemisches abzuführen. Das erfindungsgemäße Verfahren kann kontinuierlich oder intermitierend durchgeführt werden, das heißt die Zuführung des Reaktoreingangsgemisches kann mit konstantem Feed oder mit zyklisch variierender Feedzusammensetzung erfolgen. Das Gasgemisch kann an dem Katalysator in einem Festbett, beispielsweise in einem Rohrbündelreaktor oder Hordenreaktor, oder in einem Fließ- bzw. Wirbelbett reagieren. Bevorzugt sind gekühlte Rohrbündelreaktoren mit festem Katalysatorbett. Besonders bevorzugt sind Ausführungen mit zu Rohrbündel angeordneten Einzelrohren mit einem Rohrinnendurchmesser von 10 mm bis 50 mm und einer Rohrlänge von 1 m bis 6 m.

Die Strömungsgeschwindigkeit, bezogen auf das ungefüllte Rohr, in den Reaktionsrohren beträgt im allgemeinen zwischen 0,1 m/s und 10 m/s, bevorzugt zwischen 0,3 m/s und 5 m/s, besonders bevorzugt 0,5 bis 3 m/s.

Die Reaktionsrohre können mit Katalysator unterschiedlicher Zusammensetzung, Gestalt und Dimension gefüllt sein. Die Füllung kann in axialer Richtung homogen oder zonenweise variabel in die Reaktionsrohre eingebracht sein. Jede Zone kann einen statistisch verdünnten oder gemischten Katalysator enthalten.

Ein weiterer Gegenstand der Erfindung ist eine Vorrichtung zur Herstellung von gesättigten Carbonsäuren mit ein bis vier C-Atomen, nach dem erfindungsgemäßen Verfahren. Die Erfindung wird nachfolgend mit Hilfe von Figuren in schematischer Darstellung näher beschrieben.

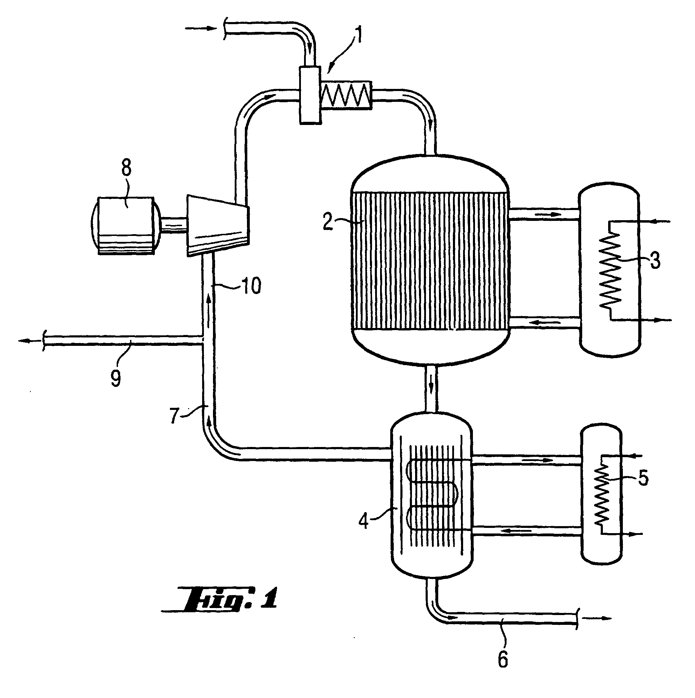

Figur 1 beschreibt eine Vorrichtung zur Herstellung von Säuren durch Gasphasenoxidation von gesättigten und/oder ungesättigten Kohlenwasserstoffen an einem Katalysator mit Kreislaufführung eines Teils des Reaktionsgases wobei die Säureabtrennung im Hauptstrom durch Kühlung und/oder Kondensation erfolgt. Hierbei werden über eine Mischzone (1) ein sauerstoffhaltiges Gas, Sauerstoff oder Luft und deren Gemische, Wasserdampf und die gesättigten und/oder ungesättigten Kohlenwasserstoffe zugeführt und vermengt und einem Rohrbündelreaktor (2) zugeführt, der mittels einer Umlaufkühlung (3) gekühlt wird. Das den Reaktor verlassende Gasgemisch wird im Hauptstrom durch einem Produktkondensator (4) geleitet, der durch einen Kühlkreislauf (5) gekühlt wird. In diesem Produktkondensator wird die Rohsäure abgetrennt und über Leitung (6) der weiteren Aufarbeitung zugeführt. Das restliche Reaktionsgas wird über Leitung (7) mittels einem Kreisgaskompressor (8) zur Mischzone zurückgeführt. Über Leitung (9) wird zur Aufrechterhaltung stationärer Bedingungen im Reaktionskreislauf ein geringer Abgasstrom entnommen. Das Abgas enthält überwiegend Kohlenoxide und nicht umgesetzte Kohlenwasserstoffe und kann einer thermischen Nutzung (Abgasverbrennung) oder sonstigen Abgasbehandlung zugeführt werden. Die Kohlenwasserstoffe können größtenteils oder auch nur teilweise aus diesem Abgasstrom beispielsweise durch Kompression und/oder Kühlung abgetrennt und als zusätzlicher Feed dem Reaktionskreislauf wieder zugeführt werden.

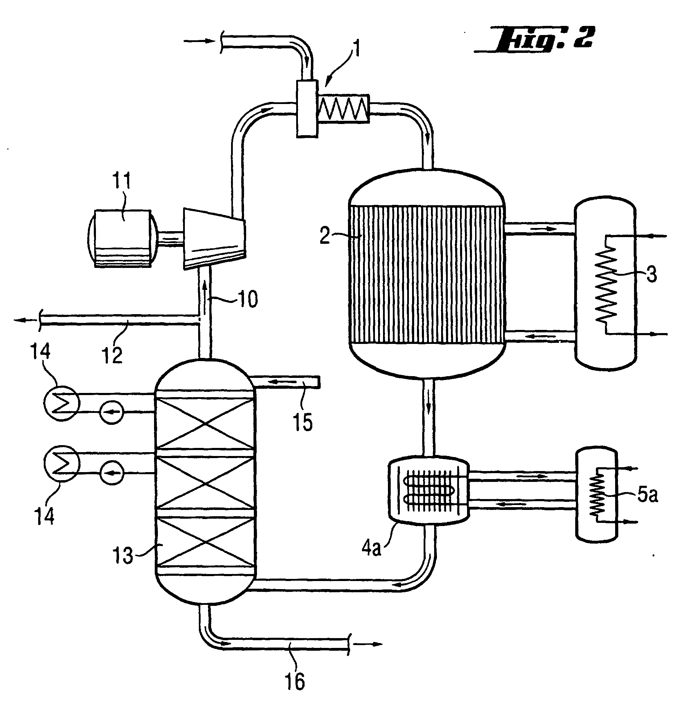

Figur 2 beschreibt eine Vorrichtung zur Herstellung von Säuren durch Gasphasenoxidation von gesättigten und/oder ungesättigten Kohlenwasserstoffen an einem Katalysator mit Kreislaufführung eines Teils des Reaktionsgases nach dem erfindungsgemäßen Verfahren. Die Abtrennung der Rohsäure wird dabei durch eine Gegenstromabsorption mittels eines geeigneten Lösungsmittels, beispielsweise Wasser, durchgeführt. Hierbei wird über eine Mischzone (1) ein sauerstoffhaltiges Gas, Sauerstoff oder Luft und deren Gemische und die gesättigten und/oder ungesättigten Kohlenwasserstoffe mit dem rückgeführten Gasstrom vermischt und mit diesem zusammen dem Rohrbündel-

5

reaktor (2) zugeführt, der mittels einer Umlaufkühlung (3) gekühlt wird. Das den Reaktor verlassende Gasgemisch wird im Hauptstrom durch einen Gaskühler (4a) geleitet der durch einen Umlaufkühler (5a) gekühlt wird. Im Anschluß daran wird das Reaktionsgas in eine Absorptionskolonne (13) geleitet, die mit einem oder mehreren Kolonnenkühlern (14) ausgerüstet ist. Im obersten Kolonnenboden wird ein geeignetes Lösungsmittel, beispielsweise Wasser, durch eine Rohrleitung (15) zugeleitet. In dieser Absorptionskolonne wird die Rohsäure durch Gegenstromwäsche abgetrennt und über ein Rohr (16) der weiteren Aufarbeitung zugeführt. Das restliche Reaktionsgas wird über ein Rohr (10) mittels einem Kreisgaskompressor (11) zur Mischzone zurückgeführt. Über Leitung (12) wird zur Aufrechterhaltung stationärer Bedingungen im Reaktionskreislauf ein geringer Abgasstrom entnommen.

Der entnommene Abgasstrom enthält überwiegend Kohlenoxide und nicht umgesetzte Kohlenwasserstoffe und kann einer thermischen Nutzung, beispielsweise einer Abgasverbrennung, einer stofflichen Nutzung oder einer sonstigen Abgasbehandlung zugeführt werden. Die Kohlenwasserstoffe können größtenteils oder auch nur teilweise aus diesem Abgasstrom durch geeignete Verfahren, beispielsweise durch Kompression oder Kühlung oder einer Kombination dieser Verfahren, verflüssigt werden und von den nicht kondensierbaren Bestandteilen abgetrennt werden und als zusätzlicher Feed dem Reaktionskreislauf, beispielsweise am Reaktoreingang, wieder zugeführt werden.

Die folgenden Beispiele dienen zur weiteren Erläuterungen der Erfindung.
Die Selektivität [in mol-%] wurde wie folgt berechnet:

$$\text{Essigsäureselektivität bezüglich gesamt } C_4\text{-Umsatz (mol-\%)}$$

$$= (((\text{mol/h Essigsäure in der Rohsäure})/2)/(\text{mol/h umgesetztes}$$

$$\text{Buten} + \text{mol/h umgesetztes Butan}))*100$$

$$\text{Ameisensäureselektivität bezüglich gesamt } C_4\text{-Umsatz (mol-\%)}$$

$$= (((\text{mol/h Ameisensäure in der Rohsäure})/4)/(\text{mol/h umgesetztes}$$

$$\text{Buten} + \text{mol/h umgesetztes Butan}))*100$$

In den Beispielen verwendete Katalysatoren:

Katalysator A: Die Herstellung des Katalysators erfolgte analog DE-A-19649426 dadurch gekennzeichnet, daß die aktive Masse aus Oxiden von Titan, Vanadium, und Antimon der empirischen Formel $Ti_aV_bSb_dO_e$ besteht (a: 125; b: 10; d: 12; e: 293) und in einem Anteil von 9 Gew.-% plus 1 Gew.-% Graphit bezogen auf das Gewicht des Trägers auf Steatitringe der Dimension 7 mm Außendurchmesser * 4 mm Innendurchmesser * 7 mm Höhe aufgebracht ist.

Katalysator B: Die Herstellung des Katalysators erfolgte analog DE-A-19649426 dadurch gekennzeichnet, daß die aktive Masse aus Oxiden von Titan, Vanadium, und Antimon der empirischen Formel $Ti_aV_bSb_dO_e$ besteht (a: 125; b: 10; d: 12; e: 293) und in einem Anteil von 10,8 Gew.-% plus 1,2 Gew.-% Graphit bezogen auf das Gewicht des Trägers auf Steatitringe der Dimension 7 mm Außendurchmesser * 4 mm Innendurchmesser * 7 mm Höhe aufgebracht ist.

Katalysator C: Die Herstellung des verwendeten Katalysators erfolgte analog DE-A 19649426 dadurch gekennzeichnet, daß die aktive Masse aus Oxiden von Titan, Vanadium, Molybdän und Antimon der empirischen Formel $Ti_aV_bWo_cSb_dO_e$ besteht (a: 122; b: 9; c: 5; d: 4; e: 288) und in einem Anteil von 14,4 Gew.-% plus 1,6 Gew.-% Graphit bezogen auf das Gewicht des Trägers auf Steatitringe der Dimension 7 mm Außendurchmesser * 4 mm Innendurchmesser * 4 mm Höhe aufgebracht ist.

Katalysator D: Die Herstellung des Katalysators erfolgte analog DE-A-19649426 dadurch gekennzeichnet, daß die aktive Masse aus Oxiden von Titan, Vanadium, und Antimon der empirischen Formel $Ti_aV_bSb_dO_e$ besteht (a: 125; b: 10; d: 12; e: 293) und in einem Anteil von 9,5 Gew.-% plus 0,5 Gew.-% Graphit bezogen auf das Gewicht des Trägers auf Steatitringe der Dimension 7 mm Außendurchmesser * 4 mm Innendurchmesser * 7 mm Höhe aufgebracht ist.

Katalysator E: Die Herstellung des Katalysators erfolgte analog DE-A-19649426 dadurch gekennzeichnet, daß die aktive Masse aus Oxiden von Titan, Vanadium, und Antimon der empirischen Formel $Ti_aV_bSb_dO_e$ besteht (a: 125; b: 10; d: 12; e: 293) und in einem Anteil von 16,2 Gew.-% plus 1,8 Gew.-% Graphit bezogen auf das Gewicht des Trägers auf Steatitringe der Dimension 7 mm Außendurchmesser * 4 mm Innendurchmesser * 7 mm Höhe aufgebracht ist.

**Vergleichsbeispiel 1 (Kreisverfahren mit Säureabtrennung nach Figur 1) :**

In einen Reaktor mit einem Reaktionsrohrinnendurchmesser von 25 mm wurde ein Katalysator des Typs A mit einer Füllhöhe von 6000 mm eingebracht. Als Reaktionsgas wurden 1000 g/h Wasserdampf, 350 g/h Sauerstoff, 150 g/h 1-Buten und 200 g/h n-Butan eingespeist. Der Kreisgasfluß wurde so eingestellt, daß der Reaktor im stabilen Zustand einen Kreisgasfluß von 11000 g/h erreichte. Der Reaktor wurde bei $11*10^5$ Pa Druck und 193°C Kühlmitteltemperatur betrieben.
Die Säureabtrennung aus dem Reaktionsgas erfolgte durch Teilkondensation bei 70°C.
Unter diesen Bedingungen wurde ein Butenumsatz von 96 % und ein Butanumsatz von 22 % erreicht. Die Essigsäureselektivität bezüglich des gesamt $C_4$-Umsatzes betrug 61 mol-%, die Ameisensäureselektivität bezüglich des gesamten C4-Umsatzes betrug 12 mol-%. Die Rohsäurekonzentration betrug 21 Gew.-%.

**Beispiel 1 (Kreisverfahren mit Säureabtrennung nach Figur 2) :**

In einen Reaktor mit einem Reaktionsrohrinnendurchmesser von 25 mm wurde ein Katalysator des Typs A mit einer Füllhöhe von 6000 mm eingebracht. Als Reaktionsgas wurden 345 g/h Sauerstoff, 144 g/h 1-Buten und 61 g/h n-Butan eingespeist. Der Kreisgasfluß wurde so eingestellt, daß der Reaktor im stabilen Zustand einen Kreisgasfluß von 10000 g/h erreichte. Der Reaktor wurde bei $11*10^5$ Pa Druck und 190°C Kühlmitteltemperatur betrieben.
Die Säureabtrennung aus dem Reaktionsgas erfolgte durch Absorption mit 600 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein Butenumsatz von 99 % und ein Butanumsatz von 65 % erreicht.-Die Essigsäureselektivität bezüglich des gesamten $C_4$-Umsatzes betrug 63 mol-%, die Ameisensäureselektivität bezüglich des gesamten $C_4$-Umsatzes betrug 13 mol-%. Die Rohsäurekonzentration betrug 31 Gew.-%.

**Beispiel 2 (Kreisverfahren mit Säureabtrennung nach Figur 2) :**

In einen Reaktor mit einem Reaktionsrohrinnendurchmesser von 25 mm wurde eine Katalysatorschicht (Oberschicht) mit 3000 mm Füllhöhe des Katalysators B, und eine weitere Katalysatorschicht (Unterschicht) mit 3000 mm Füllhöhe des Katalysators C eingefüllt. Als Reaktionsgas wurden 345 g/h Sauerstoff, 148 g/h 1-Buten und 60 g/h n-Butan zugeführt. Der Kreisgasfluß wurde so eingestellt, daß der Reaktor im stabilen Zustand einen Kreisgasfluß von 10000 g/h erreichte. Der Reaktor wurde bei $11*10^5$ Pa Druck und 190°C Kühlmitteltemperatur betrieben.
Die Säureabtrennung aus dem Reaktionsgas erfolgte durch Absorption mit 1000 g/h Wasser (Aufgabe am Kopf) in einem Absorber mit strukturierter Packung, einem Innendurchmesser von 43 mm und einer Packungshöhe von 3240 mm bei einer Kopftemperatur des Absorbers von 130°C.
Unter diesen Bedingungen wurde ein Butenumsatz von 99 % und ein Butanumsatz von 66 % erreicht. Die Essigsäureselektivität bezüglich des gesamten $C_4$-Umsatzes betrug 64 mol-%, die Ameisensäureselektivität bezüglich des gesamten $C_4$-Umsatzes betrug 13 mol-%. Die Rohsäurekonzentration betrug 23 Gew.-%.

**Beispiel 3 (Kreisverfahren mit Säureabtrennung nach Figur 2):**

In einen Reaktor mit einem Reaktionsrohrinnendurchmesser von 25 mm wurde eine Katalysatorschicht (Oberschicht) mit 3000 mm Füllhöhe des Katalysator B, und eine weitere Katalysatorschicht (Unterschicht) mit 3000 mm Füllhöhe des Katalysators C eingefüllt. Als Reaktionsgas wurden 345 g/h Sauerstoff, 148 g/h 1-Buten und 60 g/h n-Butan zugeführt. Der Kreisgasfluß wurde so eingestellt, daß der Reaktor im stabilen Zustand einen Kreisgasfluß von 10000 g/h erreichte. Der Reaktor wurde bei $11*10^5$ Pa Druck und 188°C Kühlmitteltemperatur betrieben.
Die Säureabtrennung aus dem Reaktionsgas erfolgte durch Absorption mit 2000 g/h Wasser (Aufgabe am Kopf)in einem Absorber (strukturierte Packung) mit einem Innendurchmesser von 43 mm und einer Packungshöhe von 3240 mm bei einer Kopftemperatur des Absorbers von 130°C.
Unter diesen Bedingungen wurde ein Butenumsatz von 99 % und ein Butanumsatz von 63 % erreicht. Die Essigsäureselektivität bezüglich des gesamten $C_4$-Umsatzes betrug 64 mol-%, die Ameisensäureselektivität bezüglich des gesamten $C_4$-Umsatzes betrug 13 mol-%. Die Rohsäurekonzentration betrug 13 Gew.-%.

# EP 1 035 101 B1

**Beispiel 4 (Kreisverfahren mit Säureabtrennung nach Figur 2):**

In einen Reaktor mit einem Reaktionsrohrinnendurchmesser von 25 mm wurde eine Katalysatorschicht (Oberschicht) mit 3000 mm Füllhöhe des Katalysators D, und eine weitere Katalysatorschicht (Unterschicht) mit 3000 mm Füllhöhe des Katalysators E eingefüllt. Als Reaktionsgas wurden 322 g/h Sauerstoff, 126 g/h 1-Buten und 48 g/h n-Butan zugeführt. Der Kreisgasfluß wurde so eingestellt, daß der Reaktor im stabilen Zustand einen Kreisgasfluß von 14000 g/h erreichte. Der Reaktor wurde bei $13*10^5$ Pa Druck und $193°C$ Kühlmitteltemperatur betrieben.

Die Säureabtrennung aus dem Reaktionsgas erfolgte durch Absorption mit 1000 g/h Wasser (Aufgabe am Kopf)in einem Absorber (strukturierte Packung) mit einem Innendurchmesser von 43 mm und einer Packungshöhe von 3240 mm bei einer Kopftemperatur des Absorbers von $130°C$.

Unter diesen Bedingungen wurde ein Butenumsatz von 99 % und ein Butanumsatz von 78 % erreicht. Die Essigsäureselektivität bezüglich des gesamten $C_4$-Umsatzes betrug 63 mol-%, die Ameisensäureselektivität bezüglich des gesamten $C_4$-Umsatzes betrug 13 mol-%. Die Rohsäurekonzentration betrug 21 Gew.-%.

**Beispiel 5 (Kreisverfahren mit Säureabtrennung nach Figur 2):**

In einen Reaktor mit einem Reaktionsrohrinnendurchmesser von 25 mm wurde eine Katalysatorschicht (Oberschicht) mit 3000 mm Füllhöhe des Katalysators D, und eine weitere Katalysatorschicht (Unterschicht) mit 3000 mm Füllhöhe des Katalysators E eingefüllt. Als Reaktionsgas wurden 319 g/h Sauerstoff, 126 g/h 1-Buten und 48 g/h n-Butan zugeführt. Der Kreisgasfluß wurde so eingestellt, daß der Reaktor im stabilen Zustand einen Kreisgasfluß von 16000 g/h erreichte. Der Reaktor wurde bei $15*10^5$ Pa Druck und $190°C$ Kühlmitteltemperatur betrieben.

Die Säureabtrennung aus dem Reaktionsgas erfolgte durch Absorption mit 2000 g/h Wasser (Aufgabe am Kopf)in einem Absorber (strukturierte Packung) mit einem Innendurchmesser von 43 mm und einer Packungshöhe von 3240 mm bei einer Kopftemperatur des Absorbers von $130°C$.

Unter diesen Bedingungen wurde ein Butenumsatz von 99 % und ein Butanumsatz von 78 % erreicht. Die Essigsäureselektivität bezüglich des gesamten $C_4$-Umsatzes betrug 63 mol-%, die Ameisensäureselektivität bezüglich des gesamten $C_4$-Umsatzes betrug 13 mol-%. Die Rohsäurekonzentration betrug 11 Gew.-%.

**Patentansprüche**

1. Verfahren zur Herstellung von gesättigten Carbonsäuren mit ein bis vier C-Atomen durch Gasphasenoxidation bei einer Reaktionstemperatur von $100°C$ bis $400°C$ und bei Drücken von $1,2*10^5$ Pa bis $51*10^5$ Pa, in Gegenwart von gesättigten oder ungesättigten $C_4$-Kohlenwasserstoffen und deren Gemischen, einem sauerstoffhaltigen Gas und Wasserdampf in Anwesenheit von zumindest einem Katalysator, wobei das Reaktionsausgangsgas in einem Reaktionsgaskreislauf zum Teil zurückgeführt wird und dabei durch einen Abtrennschritt die Säurekonzentration im zurückgeführten Anteil verringert wird, **dadurch gekennzeichnet, daß** die Rohsäure aus dem Reaktionsausgangsgas durch eine Gegenstromwäsche abgetrennt wird.

2. Verfahren nach Anspruch 1, **dadurch gekennzeichnet, daß** als Lösungsmittel bei der Gegenstromwäsche Verbindungen der Gruppe enthaltend Diphenyloxid, Diphenyl, aromatische- und aliphatische Ketone und Ether, Wasser, Phthalsäure und Phtalsäurederivate, Phthalid, aliphatische Dicarbonsäuren, Adipinsäure und Adipinsäurederivate, Maleinsäure und Maleinsäurederivate, Carboxyessigsäuren, Benzoesäure und Benzoesäurederivate, Lactone, Propylencarbonat, Dialkylcarbonate, Trialkylphosphate, Trialkylamine, Sulfolan und Sulfolanderivate, Alkylpyrollidone, niedermolekulare d.h. flüssige Polymere bzw. Oligomere, Polyvinylate, Polyacrylate, Polyether, Polyketone, oder Mischungen solcher Verbindungen verwendet werden.

3. Verfahren nach Anspruch 1, **dadurch gekennzeichnet, daß** als Lösungsmittel bei der Gegenstromwäsche Wasser verwendet wird.

4. Verfahren nach Anspruch 1 bis 3, **dadurch gekennzeichnet, daß** die Gegenstromwäsche in einem Absorptionsturm mit Füllkörperschüttung durchgeführt wird.

5. Verfahren nach Anspruch 1 bis 4, **dadurch gekennzeichnet, daß** die Gegenstromwäsche in einem Absorptionsturm mit geordneter Packung durchgeführt wird.

6. Verfahren nach Anspruch 1 bis 5, **dadurch gekennzeichnet, daß** die Gegenstromwäsche in einem Absorptionsturm mit einem oder mehreren Einbauten der Gruppe umfassend Trennböden, Glockenböden, Ventilböden, Siebböden oder Gitterböden durchgeführt wird.

7. Verfahren nach Anspruch 1 bis 6, **dadurch gekennzeichnet, daß** die Gegenstromwäsche in einem Riesel- oder Sprühturm durchgeführt wird.

8. Verfahren nach Anspruch 1 bis 7, **dadurch gekennzeichnet, daß** die Gegenstromwäsche in einem Absorber mit bewegten Einbauten durchgeführt wird.

9. Verfahren nach Anspruch 1 bis 8, **dadurch gekennzeichnet, daß** das bei der Konzentrierung und Reinigung der Rohsäure anfallende Wasser ganz oder teilweise in die Gegenstromwäsche zurückgeführt wird.

10. Verfahren nach Anspruch 1 bis 9, **dadurch gekennzeichnet, daß** anfallende Leichtsieder ganz oder teilweise in die Gegenstromwäsche zurückgeführt werden.

11. Verfahren nach Anspruch 1 bis 10, **dadurch gekennzeichnet, daß** die aus dem Kreisverfahren entnommenen Abgasströme thermisch oder stofflich verwertet werden.

12. Verfahren nach Anspruch 1 bis 11, **dadurch gekennzeichnet, daß** aus den Abgasströmen, die aus dem Kreisverfahren entnommen werden, durch ein oder mehrere Verfahren umfassend Kühlung oder Kompression, ein Teil der darin enthaltenen Kohlenwasserstoffe von den nicht kondensierbaren Bestandteilen abgetrennt werden und dem Reaktoreingang als Rückführungsstrom zurückgeführt werden.

13. Verfahren nach Anspruch 1 bis 12, **dadurch gekennzeichnet, daß** durch die Gegenstromwäsche die Restsäurekonzentration des wieder in den Reaktor zurückgeführten Gasstroms auf 0,01 bis 6 Vol.-% reduziert wird.

14. Verfahren nach Anspruch 1 bis 13, **dadurch gekennzeichnet, daß** der Anteil des rückgeführten Gasmassenstroms zwischen dem 1-fachen und dem 100-fachen des frisch zugeführten Eduktmassenstroms beträgt.

15. Verfahren nach Anspruch 1 bis 14, **dadurch gekennzeichnet, daß** als $C_4$-Kohlenwasserstoffe n-Butan oder die ButenIsomere 1-Buten, trans-2-Buten und cis-2-Buten sowie Gemische, die diese Verbindungen in hohen Anteilen enthalten, eingesetzt werden.

16. Verfahren nach Anspruch 1 bis 15, **dadurch gekennzeichnet, daß** als $C_4$-Kohlenwasserstoffe Rohstoffgemische aus der Petrochemie umfassend "$C_4$-Schnitt" (überwiegender Anteil an Butadien und i-Buten), "Raffinat 1" (überwiegender Anteil an i-Buten und n-Butenen) und "Raffinat 2" (überwiegender Anteil an Butanen, 1-Buten und 2-Butenen) oder Mischungen die solche Kohlenwasserstoffe enthalten, eingesetzt werden.

17. Verfahren nach Anspruch 1 bis 16, **dadurch gekennzeichnet, daß** als sauerstoffhaltiges Gas reiner Sauerstoff verwendet wird.

18. Verfahren nach Anspruch 1 bis 17, **dadurch gekennzeichnet, daß** die Sauerstoffkonzentration des dem Reaktor zugeführten Gasstroms 1 bis 35 Vol.-% beträgt.

19. Verfahren nach Anspruch 1 bis 18, **dadurch gekennzeichnet, daß** der Anteil an $C_4$-Kohlenwasserstoff am Reaktoreingang so bemessen wird, daß Buten in einer Menge von 1 bis 10 Vol.-%, allein oder im Gemisch mit weiteren $C_4$-Kohlenwasserstoffen, oder Butan in einer Menge von 5 bis 80 Vol.-%, allein oder im Gemisch mit weiteren $C_4$-Kohlenwasserstoffen vorliegt.

20. Verfahren nach Anspruch 1 bis 19, **dadurch gekennzeichnet, daß** als Katalysator ein Schalenkatalysator eingesetzt wird, bestehend aus einem inerten unporösen Trägerkörper und einer auf die äußere Oberfläche des Trägerkörpers aufgebrachten katalytisch aktiven Mischoxidmasse, welche a) ein oder mehrere Oxide aus der Gruppe Titandioxid, Zirkoniumdioxid, Zinndioxid, Aluminiumoxid und b) 0,1 bis 1,5 Gew.-%, bezogen auf das Gewicht der Komponente a) und pro $m^2/g$ spezifischer Oberfläche der Komponente a), Vanadiumpentoxid enthält.

21. Vorrichtung zur Herstellung von gesättigten Carbonsäuren mit ein bis vier C-Atomen durch Gasphasenoxidation unter ganz oder teilweiser Rückführung des Reaktionsausgangsgases und Abtrennung der Rohsäure, umfassend eine Mischzone (1) vor einem Reaktor (2) der mit einer Umlaufkühlung (3) ausgerüstet ist, wobei zur Kühlung des Reaktionsausgangsgases im Hauptstrom ein Gaskühler (4) mit Umlaufkühler (5) nachgeschaltet ist und im Kreislauf eine Leitung (12) zur Entnahme geringer Abgasströme zur Aufrechterhaltung stationärer Bedingungen im Reaktionskreislauf enthaltend ist, **dadurch gekennzeichnet, daß** die Vorrichtung zur Abtrennung der Reaktionsprodukte eine Absorptionskolonne (6) mit einem oder mehreren Kolonnenkühlern (7) umfaßt, wobei die Absorptionskolonne mit einer Rohrleitung (8) zur Zuleitung eines geeigneten Lösungsmittels für die Gegenstromwäsche, einem Rohr (9) für die Abtrennung der Rohsäure zur weiteren Aufarbeitung, und einem Rohr (10) zur Rückführung des restlichen Reaktionsgases über Pumpe (11) zur Mischzone (1) ausgerüstet ist.

22. Vorrichtung nach Anspruch 21, **dadurch gekennzeichnet, daß** als Reaktor ein Rohrreaktor/Rohrbündelreaktor eingesetzt wird.

23. Vorrichtung nach Anspruch 21 bis 22, **dadurch gekennzeichnet, daß** der Katalysator im Reaktor als Festbett vorliegt.

24. Vorrichtung nach Anspruch 21 bis 23, **dadurch gekennzeichnet, daß** die feste Katalysatorschüttung in axialer Richtung zonenweise variabel in Zusammensetzung, Gestalt und Dimension in die Reaktionsrohre eingebracht wird.

25. Vorrichtung nach Anspruch 21 bis 24, **dadurch gekennzeichnet, daß** die Absorptionskolonne eine Füllkörperschüttung beinhaltet.

26. Vorrichtung nach Anspruch 21 bis 25, **dadurch gekennzeichnet, daß** die Absorptionskolonne eine geordnete Packung beinhaltet.

27. Vorrichtung nach Anspruch 21 bis 26, **dadurch gekennzeichnet, daß** die Absorptionskolonne eine oder mehrere Einbauten der Gruppe umfassend Trennböden, Glockenböden, Ventilböden, Siebböden oder Gitterböden durchgeführt beinhaltet.

28. Vorrichtung nach Anspruch 21 bis 27, **dadurch gekennzeichnet, daß** die Absorptionskolonne ganz oder teilweise durch einen Riesel- oder Sprühturm ersetzt wird.

29. Vorrichtung nach Anspruch 21 bis 28, **dadurch gekennzeichnet, daß** die Absorptionskolonne ganz oder teilweise durch einen Absorber mit bewegten Einbauten ersetzt wird.

## Claims

1. Process for preparing saturated carboxylic acids having from one to four carbon atoms by gas-phase oxidation at a reaction temperature of from $100°C$ to $400°C$ and pressures of from $1.2 \times 10^5$ to $51 \times 10^5$ Pa in the presence of saturated or unsaturated $C_4$-hydrocarbons and mixtures thereof, an oxygen-containing gas and water vapour and in the presence of at least one catalyst, with part of the reactor outlet gas being recirculated in a reaction gas circuit and the acid concentration in the recirculated portion being reduced by means of a separation step, **characterized in that** the crude acid is separated from the reactor outlet gas by means of a countercurrent scrub.

2. Process according to Claim 1, **characterized in that** the solvent used in the countercurrent scrub is a compound selected from the group consisting of diphenyl oxide, biphenyl, aromatic and aliphatic ketones and ethers, water, phthalic acid and phthalic acid derivatives, phthalide, aliphatic dicarboxylic acids, adipic acid and adipic acid derivatives, maleic acid and maleic acid derivatives, carboxyacetic acids, benzoic acid and benzoic acid derivatives, lactones, propylene carbonate, dialkyl carbonates, trialkyl phosphates, trialkylamines, sulpholane and sulpholane derivatives, alkylpyrrolidones, low molecular weight, i.e. liquid polymers or oligomers, polyvinylates, polyacrylates, polyethers and polyketones, or a mixture of such compounds.

3. Process according to Claim 1, **characterized in that** the solvent used in the countercurrent scrub is water.

4. Process according to any of Claims 1 to 3, **characterized in that** the countercurrent scrub is carried out in an absorption tower containing random packing elements.

5. Process according to any of Claims 1 to 4, **characterized in that** the countercurrent scrub is carried out in an absorption tower containing ordered packing.

6. Process according to any of Claims 1 to 5, **characterized in that** the countercurrent scrub is carried out in an absorption tower containing one or more internal fittings selected from the group consisting of separation trays, bubble cap trays, valve trays, sieve trays or mesh trays.

7. Process according to any of Claims 1 to 6, **characterized in that** the countercurrent scrub is carried out in a trickle or spray tower.

8. Process according to any of Claims 1 to 7, **characterized in that** the countercurrent scrub is carried out in an absorber containing moving internal fittings.

9. Process according to any of Claims 1 to 8, **characterized in that** all or part of the water obtained in the concentration and purification of the crude acid is recirculated to the countercurrent scrub.

10. Process according to any of Claims 1 to 9, **characterized in that** all or part of the low boilers obtained are recirculated to the countercurrent scrub.

11. Process according to any of Claims 1 to 10, **characterized in that** the waste gas streams taken from the circulation process are utilized thermally or for their material content.

12. Process according to any of Claims 1 to 11, **characterized in that** part of the hydrocarbons present in the waste gas streams taken from the circulation process are separated from the uncondensable constituents by one or more methods comprising cooling or compression and are recirculated as recycle stream to the reactor inlet.

13. Process according to any of Claims 1 to 12, **characterized in that** the residual acid concentration in the gas stream recirculated to the reactor is reduced to from 0.01 to 6% by volume by means of the countercurrent scrub.

14. Process according to any of Claims 1 to 13, **characterized in that** the mass flow of recirculated gas is from 1 to 100 times the mass flow of fresh starting material fed in.

15. Process according to any of Claims 1 to 14, **characterized in that** the $C_4$-hydrocarbons used are n-butane or the butene isomers 1-butene, trans-2-butene and cis-2-butene or mixtures containing high proportions of these compounds.

16. Process according to any of Claims 1 to 15, **characterized in that** the $C_4$-hydrocarbons used are raw material mixtures from the petrochemicals industry encompassing "$C_4$ fraction" (predominantly butadiene and i-butene), "raffinate 1" (predominantly i-butene and n-butenes) and "raffinate 2" (predominantly butanes, 1-butene and 2-butenes) or mixtures comprising such hydrocarbons.

17. Process according to any of Claims 1 to 16, **characterized in that** the oxygen-containing gas used is pure oxygen.

18. Process according to any of Claims 1 to 17, **characterized in that** the oxygen concentration in the gas stream fed to the reactor is from 1 to 35% by volume.

19. Process according to any of Claims 1 to 18, **characterized in that** the proportion of $C_4$-hydrocarbon at the reactor inlet is calculated so that butene is present in an amount of from 1 to 10% by volume, either alone or in admixture with further $C_4$-hydrocarbons, or butane is present in an amount of from 5 to 80% by volume, either alone or in admixture with further $C_4$-hydrocarbons.

20. Process according to any of Claims 1 to 19, **characterized in that** the catalyst used is a coated catalyst comprising an inert nonporous support body and a catalytically active mixed oxide composition applied to the outer surface of the support body. The catalytically active mixed oxide composition comprises a) one or more oxides selected from the group consisting of titanium dioxide, zirconium dioxide, tin dioxide and aluminium oxide and b) from 0.1 to 1.5% by weight, based on the weight of component a) and per $m^2/g$ of specific surface area of component a), of vanadium pentoxide.

21. Apparatus for preparing saturated carboxylic acids having from one to four carbon atoms by gas-phase oxidation with recirculation of all or part of the reactor outlet gas and separation of the crude acid, comprising a mixing zone (1) upstream of a reactor (2) which is equipped with a cooling circuit (3), with a gas cooler (4) having a cooling

EP 1 035 101 B1

circuit (5) being located downstream in the main stream for cooling the reactor outlet gas and a line (12) for taking off small waste gas streams to maintain steady-state conditions in the reaction circuit being provided in the circuit, **characterized in that** the apparatus for separating off the reaction products comprises an absorption column (6) with one or more column coolers (7), where the absorption column is equipped with a pipe (8) for feeding in a suitable solvent for the countercurrent scrub, a pipe (9) for separating off the crude acid for further work-up, and a pipe (10) for recirculating the remaining reaction gas via a pump (11) to the mixing zone (1).

**22.** Apparatus according to Claim 21, **characterized in that** the reactor used is a tube reactor/multitube reactor.

**23.** Apparatus according to Claim 21 or 22, **characterized in that** the catalyst is present as a fixed bed in the reactor.

**24.** Apparatus according to any of Claims 21 to 23, **characterized in that** the fixed catalyst bed is introduced into the reaction tubes so that its composition, shape and dimensions vary zonewise in the axial direction.

**25.** Apparatus according to any of Claims 21 to 24, **characterized in that** the absorption column contains random packing elements.

**26.** Apparatus according to any of Claims 21 to 25, **characterized in that** the absorption column contains ordered packing.

**27.** Apparatus according to any of Claims 21 to 26, **characterized in that** the absorption column contains one or more internal fittings selected from the group consisting of separation trays, bubble cap trays, valve trays, sieve trays and mesh trays.

**28.** Apparatus according to any of Claims 21 to 27, **characterized in that** all or part of the absorption column is replaced by a trickle or spray tower.

**29.** Apparatus according to any of Claims 21 to 28, **characterized in that** all or part of the absorption column is replaced by an absorber having moving internal fittings.

**Revendications**

**1.** Procédé pour la production d'acides carboxyliques saturés comprenant un à quatre atomes de carbone par oxydation en phase gazeuse d'un gaz contenant de l'oxygène et de la vapeur d'eau, à une température de réaction de 100°C à 400°C et à des pressions de $1{,}2*10^5$ Pa à $51*10^5$ Pa, en présence d'hydrocarbures en $C_4$ saturés ou insaturés et de leurs mélanges, en présence d'au moins un catalyseur, le gaz sortant de la réaction étant recyclé partiellement dans un circuit de gaz réactionnel et la concentration en acide étant diminuée dans la proportion recyclée par une étape de séparation, **caractérisé en ce que** l'acide brut est séparé du gaz sortant de la réaction par un lavage à contre-courant.

**2.** Procédé selon la revendication 1, **caractérisé en ce qu'**on utilise comme solvant lors du lavage à contre-courant des composés du groupe contenant du diphényloxyde, du diphényle, des cétones et des éthers aromatiques et aliphatiques, de l'eau, de l'acide phtalique et des dérivés de l'acide phtalique, du phtalide, des acides dicarboxyliques aliphatiques, de l'acide adipique et des dérivés de l'acide adipique, de l'acide maléique et des dérivés de l'acide maléique, des acides carboxyacétiques, de l'acide benzoïque et des dérivés de l'acide benzoïque, des lactones, du carbonate de propylène, des carbonates de dialkyle, des phosphates de trialkyle, des trialkylamines, du sulfolane et des dérivés de sulfolane, des alkylpyrrolidones, des polymères ou, selon le cas, des oligomères de bas poids moléculaire, c'est-à-dire liquides, des polyvinylates, des polyacrylates, des polyéthers, des polycétones ou des mélanges de ces composés.

**3.** Procédé selon la revendication 1, **caractérisé en ce qu'**on utilise de l'eau comme solvant lors du lavage à contre-courant.

**4.** Procédé selon les revendications 1 à 3, **caractérisé en ce que** le lavage à contre-courant est réalisé dans une tour d'absorption avec des corps de remplissage en vrac.

**5.** Procédé selon les revendications 1 à 4, **caractérisé en ce que** le lavage à contre-courant est réalisé dans une tour d'absorption avec un garnissage ordonné.

6.  Procédé selon les revendications 1 à 5, **caractérisé en ce que** le lavage à contre-courant est réalisé dans une tour d'absorption présentant un ou plusieurs encastrements du groupe constitué de plateaux de séparation, de plateaux à cloches, de plateaux à clapets, de plateaux-tamis ou de plateaux à grille.

7.  Procédé selon les revendications 1 à 6, **caractérisé en ce que** le. lavage à contre-courant est réalisé dans une tour de ruissellement ou de pulvérisation.

8.  Procédé selon les revendications 1 à 7, **caractérisé en ce que** le lavage à contre-courant est réalisé dans un appareil d'absorption avec des encastrements mobiles.

9.  Procédé selon les revendications 1 à 8, **caractérisé en ce que** l'eau formée lors de la concentration et de la purification de l'acide brut est complètement ou partiellement recyclée dans le lavage à contre-courant.

10. Procédé selon les revendications 1 à 9, **caractérisé en ce que** les proportions à bas point d'ébullition formées sont recyclées complètement ou partiellement dans le lavage à contre-courant.

11. Procédé selon les revendications 1 à 10, **caractérisé en ce que** les flux d'effluent gazeux qui sont prélevés du procédé cyclique sont valorisés thermiquement ou sur le plan de la matière.

12. Procédé selon les revendications 1 à 11, **caractérisé en ce qu'**on sépare à partir des flux d'effluents gazeux qui sont prélevés du procédé cyclique, une partie des hydrocarbures qui y sont contenus, des constituants non condensables, par un ou plusieurs procédés comprenant le refroidissement ou la compression, et qu'on les recycle à l'entrée du réacteur comme flux recyclé.

13. Procédé selon les revendications 1 à 12, **caractérisé en ce que** la concentration en acide résiduel du flux de gaz à nouveau recyclé dans le réacteur est réduite par le lavage à contre-courant à 0,01 à 6% en volume.

14. Procédé selon les revendications 1 à 13, **caractérisé en ce que** la proportion du débit massique de gaz recyclé représente 1 à 100 fois le débit massique de produit de départ amené fraîchement.

15. Procédé selon les revendications 1 à 14, **caractérisé en ce qu'**on utilise comme hydrocarbures en $C_4$ du n-butane ou les isomères du butène 1-butène, trans-2-butène et cis-2-butène ainsi que des mélanges qui contiennent ces composés en des proportions élevées.

16. Procédé selon les revendications 1 à 15, **caractérisé en ce qu'**on utilise comme hydrocarbures en $C_4$ des mélanges bruts de la pétrochimie comprenant une "fraction $C_4$" (proportion principale constituée de butadiène et de i-butène), du "raffinat 1" (proportion principale constituée de i-butène et de n-butènes) et du "raffinat 2" (proportion principale constituée de butanes, de 1-butène et de 2-butènes) ou des mélanges qui contiennent ces hydrocarbures.

17. Procédé selon les revendications 1 à 16, **caractérisé en ce que** de l'oxygène pur est utilisé comme gaz contenant de l'oxygène.

18. Procédé selon les revendications 1 à 17, **caractérisé en ce que** la concentration en oxygène du flux gazeux amené dans le réacteur est de 1 à 35% en volume.

19. Procédé selon les revendications 1 à 18, **caractérisé en ce que** la proportion en hydrocarbure en $C_4$ à l'entrée du réacteur est mesurée de telle manière que le butène se trouve en une quantité de 1 à 10% en volume, seul ou en mélange avec d'autres hydrocarbures en $C_4$ ou que le butane se trouve en une quantité de 5 à 80% en volume, seul ou en mélange avec d'autres hydrocarbures en $C_4$.

20. Procédé selon les revendications 1 à 19, **caractérisé en ce qu'**on utilise comme catalyseur un catalyseur à coquille, constitué d'un corps support inerte non poreux et d'une masse d'oxyde mixte catalytiquement active appliquée sur la surface extérieure du corps support, qui contient a) un ou plusieurs oxydes du groupe dioxyde de titane, dioxyde de zirconium, dioxyde de zinc, oxyde d'aluminium et b) 0,1 à 1,5% en poids de pentoxyde de vanadium, par rapport au poids du composant a) et par $m^2/g$ de surface spécifique du composant a).

**21.** Dispositif pour la production d'acides carboxyliques saturés comprenant un à quatre atomes de carbone par oxydation en phase gazeuse avec recyclage total ou partiel du gaz sortant de la réaction et avec séparation de l'acide brut, comprenant une zone de mélange (1) avant un réacteur (2) qui est équipé d'un refroidissement par circulation (3), un refroidisseur de gaz (4) avec refroidisseur par circulation (5) étant disposé en aval pour le refroidissement du gaz sortant de la réaction dans le flux principal et une conduite (12) étant contenue dans le circuit pour le prélèvement de faibles quantités de flux d'effluent gazeux pour le maintien de conditions stationnaires dans le circuit de réaction, **caractérisé en ce que** le dispositif pour la séparation des produits de réaction comprend une colonne d'absorption (6) comprenant un ou plusieurs refroidisseurs de colonne (7), la colonne d'absorption étant équipée d'une tubulure (8) pour l'alimentation en un solvant approprié pour le lavage à contre-courant, d'un tuyau (9) pour la séparation de l'acide brut vers le traitement ultérieur et d'un tuyau (10) pour le recyclage du reste du gaz de réaction via une pompe (11) vers la zone de mélange (1).

**22.** Dispositif selon la revendication 21, **caractérisé en ce qu'**on utilise un réacteur tubulaire/réacteur à faisceau tubulaire comme réacteur.

**23.** Dispositif selon les revendications 21 à 22, **caractérisé en ce que** le catalyseur dans le réacteur se trouve sous forme de lit fixe.

**24.** Dispositif selon les revendications 21 à 23, **caractérisé en ce que** le catalyseur fixe en vrac est introduit dans les tubes de réaction de manière variable en composition, forme et dimension selon des zones dans le sens axial.

**25.** Dispositif selon les revendications 21 à 24, **caractérisé en ce que** la colonne d'absorption comporte des corps de remplissage en vrac.

**26.** Dispositif selon les revendications 21 à 25, **caractérisé en ce que** la colonne d'absorption comporte un garnissage ordonné.

**27.** Dispositif selon les revendications 21 à 26, **caractérisé en ce que** la colonne d'absorption comporte un ou plusieurs encastrements du groupe constitué de plateaux de séparation, de plateaux à cloches, de plateaux à clapets, de plateaux-tamis ou de plateaux à grille.

**28.** Dispositif selon les revendications 21 à 27, **caractérisé en ce que** la colonne d'absorption est totalement ou partiellement remplacée par une tour de ruissellement ou de pulvérisation.

**29.** Dispositif selon les revendications 21 à 28, **caractérisé en ce que** la colonne d'absorption est remplacée totalement ou partiellement par un appareil d'absorption comprenant des encastrements mobiles.

**Fig. 1**

Fig. 2